(19) Europäisches Patentamt
European Patent Office
Office européen des brevets

(11) Veröffentlichungsnummer: **0 270 965 B1**

(12) **EUROPÄISCHE PATENTSCHRIFT**

(45) Veröffentlichungstag der Patentschrift: **02.09.92**

(51) Int. Cl.⁵: **C07C 205/12**, C07C 211/52, C07C 265/12, C07C 201/12, C07C 209/36

(21) Anmeldenummer: **87117654.1**

(22) Anmeldetag: **28.11.87**

# (54) Verfahren zur Herstellung von substituierten 3,5-Dichlor-2,4-difluor-benzolen.

(30) Priorität: **11.12.86 DE 3642332**

(43) Veröffentlichungstag der Anmeldung: **15.06.88 Patentblatt 88/24**

(45) Bekanntmachung des Hinweises auf die Patenterteilung: **02.09.92 Patentblatt 92/36**

(84) Benannte Vertragsstaaten: **CH DE FR GB IT LI**

(56) Entgegenhaltungen:
**EP-A- 0 052 833**
**US-A- 3 294 629**

**CHEMICAL ABSTRACTS, Band 102, Nr. 11, 18. März 1985, Seite 546, Zusammenfassung Nr. 95355f, Columbus, Ohio, US; T. TANABE: "Preparation of tetrachloronitrobenzonitriles and related compounds"**

**CHEMICAL ABSTRACTS, Band 104, Nr. 23, 9. Juni 1986, Seite 708, Zusammenfassung Nr. 206787m, Columbus, Ohio, US; CELAMERCK G.m.b.H. UND CO., K.-G.: "Improved preparation of 3,5-dichloro-2,4-difluoronitrobenzene from 2,3,4,5-tetrachloronitrobenzene"**

(73) Patentinhaber: **BAYER AG**

**W-5090 Leverkusen 1 Bayerwerk(DE)**

(72) Erfinder: **Blank, Heinz Ulrich, Dr.**
**Am Geusfelde 35**
**W-5068 Odenthal(DE)**
Erfinder: **Ritzer, Edwin, Dr.**
**Burbachstrasse 31**
**W-5093 Burscheid(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft ein Verfahren zur Herstellung von substituierten 3,5-Dichlor-2,4-difluorbenzolen der Formel

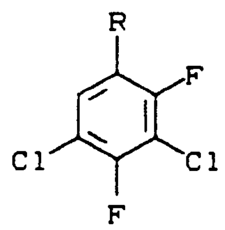

(1),

in der

R für Nitro, Amino (einschließlich der zugehörigen Ammoniumsalze) oder Isocyanato steht,

aus 2,3,4,5-Tetrachlor-nitrobenzol durch zunächst durchgeführte Umsetzung mit Alkalifluoriden bei erhöhter Temperatur in einem polaren, aprotischen Lösungsmittel, das dadurch durch gekennzeichnet ist, daß man ein rohes 2,3,4,5-Tetrachlor-nitrobenzol mit einem Gehalt von 2 bis 40 Gew-% an 2,3,5,6-Tetrachlor-nitrobenzol und untergeordnete Mengen an 2,3,4,6-Tetrachlor-nitrobenzol, bezogen auf das gesamte Tetrachlor-nitrobenzol, einsetzt und das substituierte 3,5-Dichlor-2,4-difluor-benzol auf der Nitrostufe oder nach Reduktion der Nitrogruppe auf der Aminostufe oder nach Reduktion und Phosgenierung der Aminogruppe auf der Isocyanatstufe aus dem Reaktionsgemisch isoliert.

Die Stoffe der Formel (1) sind Zwischenprodukte zur Herstellung von Wirkstoffen gegen Parasiten (US 3.294.629; EP 52 833).

Die Umsetzung von 2,3,4,5-Tetrachlor-nitrobenzol mit Kaliumfluorid zum 3,5-Dichlor-2,4-difluor-nitrobenzol in einigen polaren, aprotischen Lösungsmitteln ist bereits beschrieben worden, wenngleich die Ausbeuten häufig nicht zufriedenstellend waren; so wird in US 3.294.629 in Dimethylsulfoxid (DMSO), in EP 52 833 und in Gunma Journal of Liberal Arts and Sciences (Universität Gunma, Japan) 18 (1984), 55 - 66 in Dimethylformamid (DMF) und in Research Disclosure RD 25 517 in Dimethylsulfon ($DMSO_2$) gearbeitet.

Alle genannten Verfahren gehen von isomerenreinem 2,3,4,5-Tetrachlor-nitrobenzol aus. Die Isolierung eines reinen 2,3,4,5-Tetrachlor-nitrobenzol aus einem rohen, technisch hergestellten Tetrachlor-nitrobenzol ist jedoch kostenaufwendig und verlustreich, da die Verunreinigungen chemisch ähnliche Substanzen sind, die einen hohen Trennaufwand erfordern.

Es war daher sehr wünschenswert, ein rohes, technisch hergestelltes 2,3,4,5-Tetrachlor-nitrobenzol einzusetzen und das 3,5-Dichlor-2,4-difluor-nitrobenzol in einer angestrebten, der weiteren Verwendung angemessenen Reinheit zu erhalten.

Rohes Tetrachlor-nitrobenzol kann durch Chlorieren von Benzol oder teilweise chlorierten Benzolen zur Tetrachlorbenzol-Stufe und anschließendes Nitrieren der Tetrachlorbenzol-Fraktion mit Nitriersäure oder durch Chlorieren von Nitrobenzol oder ungenügend chlorierten Nitrobenzolen zur Tetrachlor-nitrobenzol-Stufe gewonnen werden. Ausgangsstoffe können demnach sein: Benzol, Chlorbenzol, die isomeren Dichlorbenzole und Trichlorbenzole zur weiteren Chlorierung und Nitrierung; rohes 1,2,3,4-Tetrachlorbenzol zur Nitrierung; Nitrobenzol, die isomeren Chlor-nitrobenzole, Dichlor-nitrobenzole und Trichlor-nitrobenzole zur weiteren Chlorierung. Ausgenommen ist der Einsatz von Dichlor- und Trichlor-nitrobenzolen, in denen beide ortho-Positionen zur Nitrogruppe besetzt sind. Jedoch muß in Kauf genommen werden, daß solche unerwünschten Isomeren bei der Verarbeitung technischer Gemische mitentstehen. Ähnliches gilt für die unerwünschten Polychlorbenzol-Isomeren.

Rohes 2,3,4,5-Tetrachlor-nitrobenzol ist daher vor allem durch einen Gehalt an 2,3,5,6-Tetrachlor-nitrobenzol und/oder 2,3,4,6-Tetrachlor-nitrobenzol gekennzeichnet. Mit diesen oder mit ihren Umsetzungsprodukten als unerwünschten Nebenprodukten muß daher im erfindungsgemäßen Verfahren gerechnet werden; sie lassen sich durch die allgemeinen Formeln

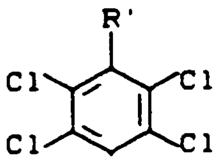 (2) bzw. 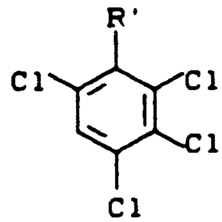 (3)

darstellen, in denen

R′ die Bedeutung von R hat und zusätzlich für Fluor stehen kann.

Während unerwünschte Nebenprodukte der Formel (2) beim Einsatz von rohem Tetrachlor-nitrobenzol auftreten, das nach beiden oben geschilderten Herstellungswegen erhalten wird, treten Nebenprodukte der Formel (3) vorwiegend beim Einsatz von rohem Tetrachlor-nitrobenzol auf, das durch Chlorieren von Nitrobenzol oder ungenügend chlorierten Nitrobenzolen erhalten wird. Daneben kann mit unvollständig umgesetzten Vorstufen im rohen Tetrachlor-nitrobenzol gerechnet werden.

Beispielhaft sei anhand des beigefügten Formelschemas auf rohes Tetrachlor-nitrobenzol über die Tetrachlor-benzol-Stufe und dessen Reaktionsprodukte eingegangen:

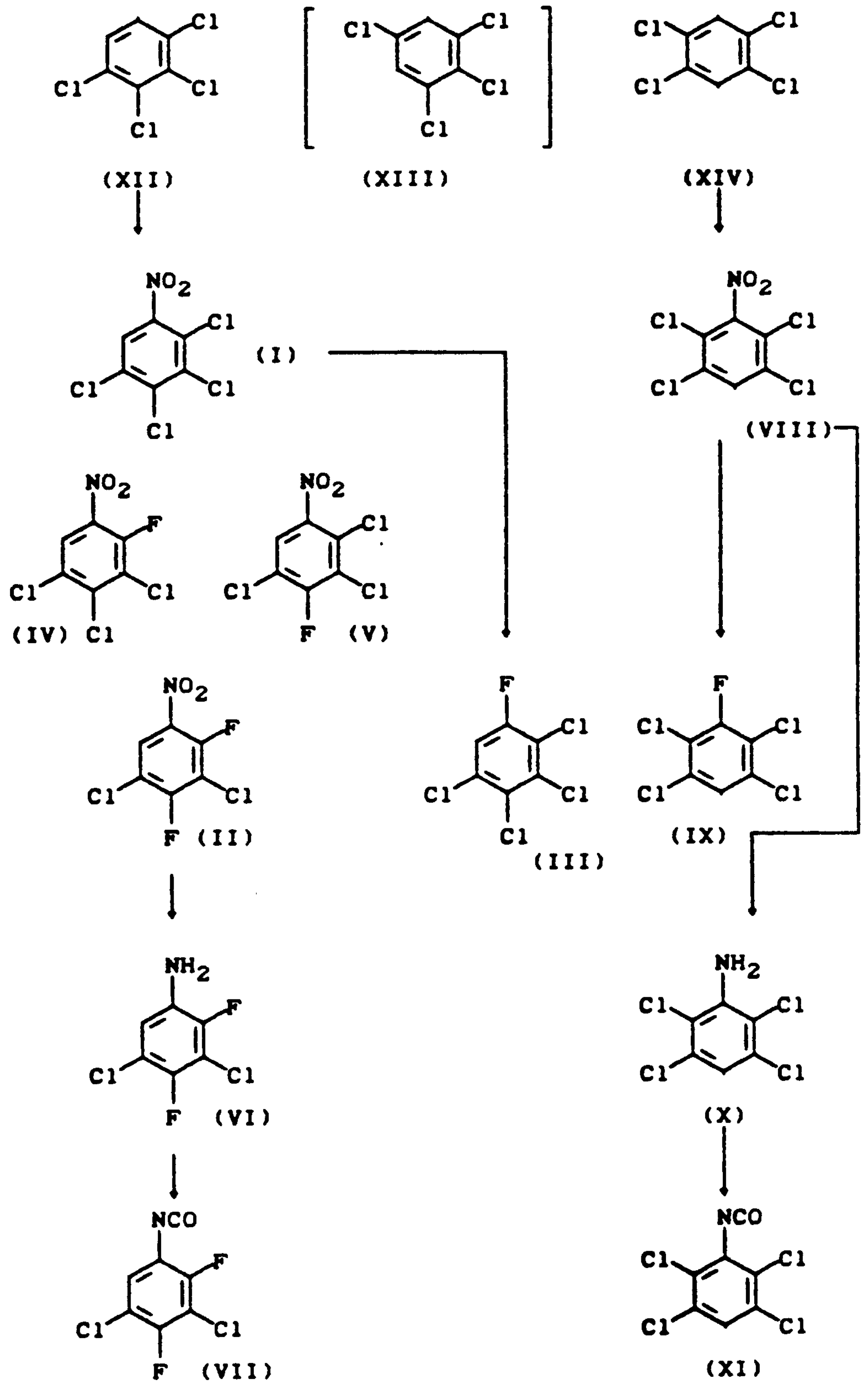

(XII) (XIII) (XIV)

(I) (VIII)

(IV) (V)

(II) (III) (IX)

(VI) (X)

(VII) (XI)

Die Tetrachlorbenzol-Fraktion enthält neben dem gewünschten 1,2,3,4-Tetrachlorbenzol (XII) unterschiedliche, jedoch störende Mengen an 1,2,4,5-Tetrachlorbenzol (XIV), und untergeordnete Mengen an 1,2,3,5-Tetrachlorbenzol (XIII). Die Menge des 1,2,4,5-Tetrachlorbenzols (XIV) schwankt zwischen 2 und 40 Gew.-% des gesamten rohen Tetrachlorbenzols. Die Tetrachlorbenzol-Fraktion enthält ferner geringe Mengen an Pentachlorbenzol und Hexachlor-benzol sowie evtl. Spuren von Trichlorbenzol.

Die genannten Verunreinigungen schlagen auf die Nitrostufe durch. Im Sinne des oben Gesagten hat

man hierbei hauptsächlich mit dem gewünschten 2,3,4,5-Tetrachlor-nitrobenzol (I) und der Hauptverunreinigung 2,3,5,6-Tetrachlor-nitrobenzol (VIII) zu rechnen.

Bei der nucleophilen Substitutionsreaktion mit Alkalifluorid erhält man aus den Verbindungen (I) und (VIII) der Nitrierstufe ein Gemisch aus dem gewünschten 3,5-Dichlor-2,4-difluor-nitrobenzol (II), den unvollständig fluorierten Verbindungen 3,4,5-Trichlor-2-fluor-nitrobenzol (IV) und 2,3,5-Trichlor-4-fluor-nitrobenzol (V), dem durch unerwünschten Fluoraustausch gebildeten 2,3,4,5-Tetrachlor-fluorbenzol (III), dem aus (VIII) hervorgegangenen 2,3,5,6-Tetrachlor-fluorbenzol (IX) und unverändertem (I) und (VIII).

Die Auftrennung eines solchen Gemisches ist wirtschaftlich nicht mehr sinnvoll durchführbar. Bei einer destillativen Trennung mit längerer thermischer Belastung ist bei so hoch substituierten Stoffen wie den genannten auch mit erheblicher Zersetzung zu rechnen.

Das erfindungsgemäße Verfahren beruht nun auf einer Reihe überraschender Befunde:

a) Das gewünschte (II) erleidet bei längerer thermischer Behandlung, als zur Erreichung der maximalen Ausbeute erforderlich ist, eine Zersetzung. Die dabei ablaufenden Vorgänge sind im einzelnen nicht bekannt; möglicherweise liegt ein autokatalytischer Ablauf vor.

b) Die unerwünschten Inhaltsstoffe 2,3,5,6- und 2,3,4,6-Tetrachlor-nitrobenzol ((VIII) und sein Stellungsisomeres mit ebenfalls 2 zur Nitrogruppe ortho-ständigen Chlorsubstituenten) im rohen Tetrachlornitrobenzol unterliegen gleichzeitig zur Umwandlung von (I) in (II) einem nucleophilen $NO_2$-F-Austausch zu den 2,3,5,6- und 2,3,4,6-Tetrachlor-fluorbenzolen ((IX) und sein Stellungsisomeres). Dieser $NO_2$-F-Austausch ("Verkochen" der genannten Nitrobenzole) verläuft grundsätzlich langsamer als die gewünschte Umwandlung von (I) in (II).

c) Überraschenderweise ergab sich, daß die thermische Zersetzung nach a) zunächst jedoch nur langsam anläuft und erst nach längerer Erhitzungsdauer einen stärkeren Umfang annimmt, so daß ein "Verkochen" nach b) vorgenommen werden kann, um die Verunreinigung durch die unerwünschten genannten 2,3,5,6-bzw. 2,3,4,6-Tetrachlor-nitrobenzole auf ein tolerierbares Maß zu senken, ohne gleichzeitig zu hohe Ausbeuteverluste an (II) hinnehmen zu müssen.

d) Was beim rohen Tetrachlor-nitrobenzol nicht wirtschaftlich sinnvoll möglich war, ist nunmehr nach der nucleophilen Fluorierung möglich, nämlich eine physikalische Abtrennung der Verunreinigungen, hauptsächlich in Form der $NO_2$-freien Fluorbenzole.

e) In weiterer Ausgestaltung des erfindungsgemäßen Verfahrens, auf die weiter unten näher eingegangen wird, wurde gefunden, daß sich die Abtrennung mit physikalischen Methoden auch nach Reduktion der Nitrogruppe auf der Aminostufe (VI) oder nach Reduktion und Phosgenierung auf der Isocyanatstufe (VII) erfolgreich durchführen läßt, wobei auch eine Kombination von "Verkochen" und einer physikalischen Trennung erst auf einer der Folgestufen möglich ist. Der jeweils zu beschreitende Weg ist abhängig vom Grad der Verunreinigung des rohen Tetrachlor-nitrobenzols und vom gewünschten Grad an restlicher Verunreinigung in (II) bzw. (VI) bzw. (VII).

Zur Erkennung der unter a) bis d) genannten Befunde und zur Durchführung des erfindungsgemäßen Verfahrens bedient man sich üblicher analytischer Methoden, z.B. der Gaschromatographie. Dabei kann sich herausstellen, daß bereits bei Erreichen der maximalen Ausbeute an (II) eine genügende Absenkung des Gehalts an den genannten unerwünschten Tetrachlor-nitrobenzolen eingetreten ist, daß eine physikalische Abtrennung zum gewünschten Ergebnis führt. In anderen Fällen wird die Erhitzungsdauer des Reaktionsansatzes verlängert und bei Erreichen der angestrebten, analytisch erkennbaren weiteren Verringerung der Verunreinigungen abgebrochen, wobei gegebenenfalls eine Abtrennung der restlichen Verunreinigungen bereits auf der Nitrostufe ausreichend sein kann.

Bei regelmäßiger Durchführung des erfindungsgemäßen Verfahrens mündet die analytische Beobachtung des Reaktionsansatzes selbstverständlich in standardisierte Verfahrensparameter ein, wie dies auch sonst in der chemischen Technik üblich ist.

Die nucleophile Fluorierung wird mit einem Alkalifluorid, bevorzugt einem schweren Alkalifluorid, wie Kaliumfluorid, Rubidiumfluorid oder Cäsiumfluorid, besonders bevorzugt mit Kaliumfluorid, durchgeführt. Dem Alkalifluorid kann ein an sich bekannter Zusatz von Alkalichlorid, beispielsweise 0,5 - 20 Gew.-%, bezogen auf die Menge des Alkalifluorids, zugesetzt werden. In bevorzugter Weise wird ohne einen solchen Zusatz gearbeitet.

Im erfindungsgemäßen Verfahren können ferner ein oder mehrere Phasentransfer-Katalysatoren, beispielsweise in einer Menge von 0,3 - 30 Gew.-%, bezogen auf das umzusetzende Tetrachlor-nitrobenzol, zugesetzt werden, wie Tetrabutylammoniumbromid, Trimethylphenylammoniumchlorid, Triethylbenzylammoniumchlorid, Trimethylbenzylammoniumchlorid, Hexadecyltributylphosphoniumbromid, Kronenether (18-Krone-6) u.a..

Als polares, aprotisches Lösungsmittel kommt beispielsweise in Frage: Dimethylsulfoxid (DMSO), Dimethylformamid (DMF), Dimethylsulfon ($DMSO_2$), Tetramethylensulfon ($TMSO_2$; Sulfolan), Acetonitril,

Benzonitril, Nitrobenzol, Dimethylacetamid, N-Methyl-pyrrolidon (NMP), N-Methyl-$\epsilon$-caprolactam, Tetramethyl-harnstoff, Hexamethylphosphorsäuretriamid, Diethylenglykol-dimethylether (Diglyme) und andere dem Fachmann bekannte Verbindungen.

Diese Lösungsmittel können auch als Gemisch eingesetzt werden. Ferner können bis zu 50 Gew.-%, bezogen auf die Gesamtmenge an Lösungsmittel, andere inerte Lösungsmittel, wie Benzol, Toluol, Chlorbenzol, Dichlorbenzol, Trichlorbenzol oder Tetrachlorbenzol zugesetzt werden. In bevorzugter Weise wird in DMSO, DMF oder $DMSO_2$, in besonders bevorzugter Weise in DMSO, gearbeitet.

Für den Fall, daß der Siedepunkt des abderen inerten Lösungsmittels bei Normaldruck niedriger liegt als die angestrebte Reaktionstemperatur, kann auch bei erhöhtem Druck, beispielsweise bei 1,5 - 10 bar, gearbeitet werden. Durch das Arbeiten bei vermindertem Druck kann man die Temperatur sehr gut durch das Einstellen der entsprechenden Siedegleichgewichte regulieren ("Siedekühlung"). In den meisten Fällen ist jedoch das Arbeiten bei Normaldruck ausreichend.

Das erfindungsgemäße Verfahren wird bei einer Temperatur von 60 - 160 °C, bevorzugt 80 - 140 °C, besonders bevorzugt 95 - 125 °C, durchgeführt. Die Reaktionszeit beträgt 20 - 0,2 Stunden, wobei man bei Einstellen einer tieferen Temperatur mit längeren Reaktionszeiten rechnen muß und umgekehrt. Die oben erwähnten optimierten Temperatur-Zeit-Zusammenhänge für die Bildung von (II) sind für die angegebenen Lösungsmittel etwas unterschiedlich, können aber vom Fachmann durch einfache Vorversuche bestimmt werden. So wurde beispielsweise gefunden, daß für das Lösungsmittel DMSO Temperatur und Zeit durch die Beziehung T (°C) = A -33 log t(h) verknüpft sind, worin A Werte von 100 - 137, bevorzugt 110 - 135, besonders bevorzugt 115 - 130, annimmt. Diese Mindestzeit für das erfindungsgemäße Verfahren kann je nach gewünschtem Grad an restlicher Verunreinigung verlängert werden.

Durch die analytische Verfolgung des Reaktionsansatzes, insbesondere über die zur Bildung von (II) optimierten Zeit bei vorgebener Temperatur hinaus, wird der Grad an restlicher Verunreinigung an (VIII) erkannt, so daß bei wiederholter Durchführung standardisierte Verfahrensparameter, insbesondere Temperatur und Zeit, erarbeitet werden können.

Das Verhältnis von Tetrachlor-nitrobenzol zum Alkalifluorid und zum polaren aprotischen Lösungsmittel reicht im erfindungsgemäßen Verfahren allgemein von 1:2:1 bis 1:6:30, bevorzugt von 1:2,2:2,4 bis 1:4,4:10. Bei der Verwendung von DMSO können Werte im unteren Teil des Bereiches, nämlich von 1:2,2:2,0 bis 1:5:24, bevorzugt 1:2,3:2,4 bis 1:4,0:10, besonders bevorzugt von 1:2,4:2,8 bis 1:3,2:4,6, eingehalten werden. Diese günstigen Werte sind in Richtung auf höhere Feststoffgehalte in der Suspension in DMSO nur durch die abnehmende Rührbarkeit der Suspension begrenzt. Durch so hohe Feststoffgehalte kann eine erhebliche gesteigerte Raum-Zeit-Ausbeute und eine beträchtliche Energieeinsparung erzielt werden.

Das Alkalifluorid und das eingesetzte polare, aprotische Lösungsmittel werden in wasserfreier Form eingesetzt. Hierzu kann das Alkalifluorid durch Sprühtrocknung vorbehandelt werden oder einige Zeit bei bis zu 600 °C getrocknet werden. Das Lösungsmittel wird in bekannter Form über Phosphorpentoxid oder anderen bekannten Trockenmitteln getrocknet. Soweit möglich, kann das Lösungsmittel auch durch bloße Destillation vom Wasser befreit werden; eine weitere Möglichkeit besteht darin, Toluol oder einen anderen Azeotropbildner zuzusetzen und das Wasser als Azeotrop abzudestillieren.

Bei der besonders bevorzugten Verwendung von DMSO als dem polaren, aprotischen Lösungsmittel sind in besonders günstiger Weise keine sehr scharfen Trocknungsmethoden erforderlich. So ist beispielsweise ein Alkalifluorid, das im Trockenschrank bei 200 °C/200 Torr getrocknet wurde, einsetzbar; sogar die Verwendung von handelsüblich trockenen Alkalifluoriden ist möglich.

Im allgemeinen werden das polare, aprotische Lösungsmittel oder ein Gemisch mit einem solchen Lösungsmittel und das Alkalifluorid vorgelegt und, falls dies erforderlich ist, einer Trocknung durch Azeotropdestillation unterworfen. Hierbei wird vielfach bereits die angestrebte Reaktionstemperatur erreicht. Das rohe 2,3,4,5-Tetrachlor-nitrobenzol wird dann zur Suspension des Alkalifluorids im polaren, aprotischen Lösungsmittel gegeben; diese Zugabe kann nach Erreichen der Reaktionstemperatur oder bereits vor oder während der Aufheizphase erfolgen. Soweit vor der analytischen Verfolgung des Reaktionsansatzes eine für die Bildung von (II) optimierte Reaktionszeit bei der gewählten Reaktionstemperatur durchlaufen wird, beginnt die Reaktionszeit mit dem Erreichen der Reaktionstemperatur. Bei größeren Ansätzen in technischem Umfan kann es wünschenswert sein, den Ausgangsstoff in Portionen zuzugeben; in jedem Fall benötigt die Zugabe einer größeren Menge eines Ausgangsstoffes eine angemessene, dem Fachmann aus der chemischen Verfahrenstechnik bekannte Handhabungszeit. In einem solchen Fall wird der Beginn der Reaktionszeit auf das Ende der Zugabe gesetzt.

Nach Beendigung der nucleophilen Fluorierung kann das entstandene Reaktionsgemisch in verschiedener Weise aufgearbeitet werden. So werden im allgemeinen nach Abkühlen des Reaktionsgemisches zunächst die anorganischen Salze (Alkalimetallfluoride/-chloride) abgetrennt, z.B. abfiltriert oder abzentrifugiert. Die weitere Auftrennung in Lösungsmittel und die Umsetzungsprodukte kann dann durch Destillation,

Extraktion oder durch andere physikalische Trennverfahren, wie Säulenchromatographie durchgeführt werden. Ferner kann die Lösung der fluorierten Umsetzungsprodukte im polaren, aprotischen Lösungsmittel mit Wasser versetzt werden, wobei sich die fluorierten Umsetzungsprodukte abscheiden, abgetrennt werden können und einer gegebenenfalls weiteren Aufarbeitung und Reinigung zugeführt werden können.

In einer besonders vorteilhaften Variante wird die Lösung der fluorierten Umsetzungsprodukte im polaren, aprotischen Lösungsmittel diskontinuierlich oder kontinuierlich, in bevorzugter Weise kontinuierlich, mit einem oder mehreren geradkettigen oder verzweigten, offenkettigen oder cyclischen aliphatischen Kohlenwasserstoffen, die einen Siedepunkt von mindestens 30 °C haben, extrahiert. Hierbei treten die fluorierten Umsetzungsprodukte in die Phase des aliphatischen Kohlenwasserstoffes über und können daraus durch mindestens teilweises Abdampfen des aliphatischen Kohlenwasserstoffes gewonnen werden und gegebenenfalls einer weiteren Feinreinigung zugeführt werden. Das durch Extraction von den fluorierten Umsetzungsprodukten befreite polare, aprotische Lösungsmittel kann als solches ohne weitere Aufarbeitung dem nächsten Reaktionsansatz im erfindungsgemäßen Verfahren zugeführt werden. Geeignete Extraktionsapparate für diese spezielle Verfahrensvariante sind dem Fachmann bekannt, beispielsweise Extraktoren für spezifisch leichtere Extraktionsmittel nach Ludwig (DE-AS 22 21 554). Aliphatische Kohlenwasserstoffe für diese Verfahrensvariante sind beispielsweise Pentan, Hexan, Octan, Decan, Dodecan, Hexadodecan, Cyclohexan, Methylcyclohexan, Methylcyclopentan, Isooctan, Gemische dieser aliphatischen Kohlenwasserstoffe sowie die aliphatischen Destillationsschnitte Petrolether mit Siedebereichen 30 - 50 °C, ca. 40 °C, 40 - 60 °C, 60 - 70 °C, 40 - 80 °C, Leichtbenzin (60 - 95 °C), Ligroin (80 - 110 °C), Lötbenzin (60 - 140 °C), Waschbenzin (100 - 140 °C) und andere.

Die fluorierten Umsetzungsprodukte können nach der Abtrennung vom polaren, aprotischen Lösungsmittel physikalisch aufgetrennt werden. Solche physikalischen Methoden sind die Destillation, gegebenenfalls unter vermindertem Druck, die fraktionierte Kristallisation und die Säulenchromatographie. Insbesondere durch das letztere Verfahren ist es möglich, Stoffe ohne Nitrogruppe, wie (III) und (IX) von den Nitroprodukten abzutrennen. Das gewünschte (II) kann anschließend von ungewünschten Tetrachlor-nitrobenzolen auf üblichem Weg, z.B. durch Destillation, abgetrennt werden.

Die Weiterverarbeitung von (II) zu Wirkstoffen gegen Parasiten erfolgt über die aus der Nitroverbindung hervorgehende Aminoverbindung (VI). Hierzu wird (II) beispielsweise in Lösung in den aliphatischen Kohlenwasserstoffen aus der Extraktion oder nach anderer Isolierung von (II) auch in Alkoholen oder anderen geeigneten, dem Fachmann bekannten Lösungsmitteln katalytisch an Edelmetallkontakten, Raney-Metallen oder anderen Hydrierkatalysatoren hydriert. Grundsätzlich ist auch beispielsweise eine Reduktion der Nitrogruppe mit Eisen/Säure zur Aminogruppe möglich. Die im Hydriergemisch vorhandenen Verbindungen ohne Nitrogruppe, beispielsweise (III) und (IX), verhalten sich hierbei inert. Nach beendeter Hydrierung wird sodann die Aminoverbindung (VI) mit den restlichen tolerierbaren Mengen an Verunreinigung durch Tetrachlor-aniline durch Zusatz einer Mineralsäure, wie Schwefelsäure, Phosphorsäure oder Halogenwasserstoff, bevorzugt Halogenwasserstoff, als Salz ausgefällt und kann damit von Verbindungen ohne Aminogruppe, wie (III) und (IX), abgetrennt werden. Damit entfällt die im Zusammenhang mit der Nitroverbindung (II) beschriebene Säulenchromatographie, was eine weitere Kosteneinsparung darstellt.

Ein so hergestelltes Gemisch aus (VI) und Tetrachloranilinen kann weiterhin durch die obengenannten, physikalischen Methoden, beispielsweise durch Vakuumdestillation, feingereinigt werden, wozu die Salze gegebenenfalls wieder in die freien Anilinverbindungen übergeführt werden. Die beispielsweise destillative Trennung dieser nur noch wenigen Verbindungen ist wesentlich erfolgversprechender und damit kostengünstiger als eine Anwendung physikalischer Methoden auf einer der vorangegangenen Stufen mit einem Gemisch aus vielen Verbindungen. Eine solche Feindestillation kann mit der erfindungsgemäße eintretenden Umwandlung von 2,3,5,6- bzw. 2,3,4,6-Tetrachlor-nitrobenzolen in die zugehörigen Tetrachlor-fluorbenzole derart kombiniert werden und stellt somit eine interessante Ausgestaltung des erfindungsgemäßen Verfahrens dar, daß nur ein Teil dieser Tetrachlor-nitrobenzole zu den Tetrachlor-fluorbenzolen "verkocht" wird, während ein anderer Teil auf der Anilinstufe destillativ abgetrennt wird. Insbesondere, wenn große Mengen an 2,3,6,6- bzw. 2,3,4,6-Tetrachlor-nitrobenzole zu entfernen sind, wird man bevorzugt nur einen kleineren Teil hiervon durch "Verkochen" entfernen und den größeren Teil auf der Anilinstufe oder auf einer weiteren der oben erwähnten Folgestufen destillativ abtrennen, während bei relativ kleinen Mengen an 2,3,5,6- bzw. 2,3,4,6-Tetrachlor-nitrobenzolen diese durch das erwähnte "Verkochen" allein auf ein tolerierbares Maß gesenkt werden. Hierdurch kann man in Abhängigkeit vom Grad der Verunreinigung des rohen Tetrachlornitrobenzols den gewünschten zulässigen Grad an restlicher Verunreinigung einstellen, ohne daß eine zu starke Zersetzung von (II) und damit Ausbeuteminderung in Kauf genommen werden muß.

Die bei der Abtrennung der Aniline (VI) und (X) über deren Salzform zurückbleibenden Tetrachlorfluorbenzole (III) und (IX) können separat gewonnen werden und einer eigenen Verwendung zugeführt werden.

In einer sehr ähnlichen Weise kann das Gemisch der abgetrennten Aniliniumsalze in einer dem Fachmann grundsätzlich bekannten Weise phosgeniert werden; das daraus resultierende Gemisch des Isocyanats (VII) mit Tetrachlorphenylisocyanaten kann sodann durch die obengenannten physikalischen Methoden, beispielsweise destillativ in die genannten Isocyanate getrennt werden, die eine höhere Reinheit als vorher aufweisen. Auch bei dieser Variante läßt sich also ein Teil des abgestrebten Grades an restlicher Verunreinigung durch "Verkochen" erreichen, während die weitere Absenkung an restlicher Verunreinigung beispielsweise destillativ auf der Isocyanatstufe erreicht wird.

Beispiel 1

In allen Ausführungsbeispielen beziehen sich die mit römischen Ziffern angegebenen Verbindungen auf das weiter vorne befindliche Formelschema.

Es wurde mit einem rohen Tetrachlor-nitrobenzol gearbeitet, das 78,0 % (I) und 9,2 % (VIII) enthielt; der Rest bestand im wesentlichen aus Tetrachlorbenzol, Pentachlorbenzol und Hexachlorbenzol. 26,1 g dieses rohen Tetrachlor-nitrobenzols wurden mit 15,1 g handelsüblichem, nicht weiter vorbehandeltem Kaliumfluorid und 39 g DMSO auf 120 °C erhitzt und in den in der Tabelle angegebenen Zeitabständen analytisch verfolgt, indem gezogene Proben gaschromatographisch auf ihre Zusammensetzung untersucht wurden.

**Tabelle**

**Gaschromatographisch ermittelte Zusammensetzung des Reaktionsgemisches (Gew.-%)**

| t (h) | II | III | IX | VIII | I |
|---|---|---|---|---|---|
| 1 | 35,0 | 3,9 | 2,4 | 8,4 | 16,0 |
| 2 | 54,0 | 4,9 | 4,1 | 6,9 | 5,5 |
| 4 | 61,5 | 5,1 | 5,2 | 5,7 | 2,2 |
| 6 | 65,6 | 5,9 | 8,2 | 3,0 | 1,1 |
| 8 | 59,5 | 6,6 | 10,2 | 0,9 | 0,9 |
| 12 | 54,9 | 7,1 | 10,9 | 0,8 | 1,3 |
| 16 | 51,4 | 7,9 | 11,9 | 1,0 | 1,7 |
| 20 | 47,9 | 8,3 | 12,8 | 0,8 | 1,7 |

Die zu 100 Gew.-% fehlenden Anteile verteilen sich auf Tetrachlorbenzol, Pentachlorbenzol und Hexachlorbenzol, sowie bei kürzeren Reaktionszeiten auf nur teilweise umgesetztes (IV) und (V), sowie weiterhin bei langen Reaktionszeiten auf unbekannte Zersetzungsprodukte. Diese bei längerer Reaktionszeit verstärkte Zersetzung läßt bei absolut kleiner werdender Menge an (II) und den gesamten Umsetzungsprodukten die Menge an (III) sowie die Summe aus (VIII) und (IX) relativ ansteigen.

Bei einer Wiederholung des beschrieben Versuchs wurde die Reaktion nach 8 Stunden Laufzeit unterbrochen, und die dabei erhaltenen Umsetzungsprodukte wurden mit kaltem Hexan in einem 300 ml-Rotations-Perforator nach Ludwig zur Flüssig-Flüssig-Extraktion mit spezifisch leichteren Lösungsmitteln (DE-AS 22 21 554, Firma Normag) extrahiert. Der erhaltenen Hexanphase wurde nach einmaligem Waschen mit 100 ml Wasser 2 g Raney-Nickel-Eisen als Hydrierkatalysator zugesetzt. Anschließend wurde bei 50 °C und einem $H_2$-Druck von 10 bar bis zum Ende der Wasserstoffaufnahme hydriert. Nach dem Abfiltrieren des Hydrierkatalysators wurde in die Hexanphase HCl-Gas eingeleitet, woraufhin sich (VI) als Hydrochlorid kristallin abschied. Nach Filtration und nach Waschen mit kaltem n-Hexan wurden 17,7 g (VI) mit einer Reinheit von 99 % erhalten.

Beispiel 2

Es wurde mit einem an (I) 82 gew.-%igem rohen Tetrachlornitrobenzol gearbeitet (der Rest zu 100 % bestand zu 8,9 Gew.-% aus (VIII) und sonst im wesentlichen aus Tetra-, Penta- und Hexachlorbenzol). 130 g dieses rohen Tetrachlor-nitrobenzols wurden mit 75,5 g KF in 150 g DMSO suspendiert und 4 Stunden auf 120 ° C erwärmt. Nach dem Abkühlen auf Raumtemperatur wurde das KF/KCl-Feststoffgemisch auf einer Saugnutsche abfiltriert und zweimal mit je 30 ml DMSO gewaschen. Anschließend wurde die erhaltene DMSO-Lösung in der in Beispiel 1 beschriebenen Weise mit n-Hexan extrahiert. Das Umsetzungsgemisch enthielt nach gaschromatographischer Analyse

71,0 % (II)
5,5 % (III)
6,3 % (IX)
1,4 % Pentachlorbenzol
2,8 % (VIII)
0,5 % (I).

Beispiel 3

130 g des in Beispiel 2 eingesetzten rohen Tetrachlor-nitrobenzols wurden mit 75,5 g KF in 200 g $DMSO_2$ 16 Stunden auf 135 ° C erwärmt. Anschließend ließ man auf 120 ° C abkühlen und setzte langsam 250 ml Toluol zu; hierbei sand die Temperatur weiter und $DMSO_2$ kristallisierte aus. Nach einer Stunde weiterem Abkühlen auf 10 ° C saugte man die Feststoffe ($DMSO_2$, KF, KCl) ab und wusch zweimal mit je 100 ml 0 ° C kaltem Toluol nach. Der Filterkuchen wurde zweimal mit je 100 ml 0,1 n HCl (ca. 3,7 gew.-%ig) und zweimal mit je 100 ml 3 %iger wäßriger Sodalösung gewaschen. Die Toluolphase wurde im Vakuum bis zum Rückstand eingeengt. Man erhielt 94,8 g eines Öls als Rohprodukt. Die gaschromatographische Analyse ergab folgende Gehalte:

73,1 % (II)
3,6 % (III)
8,2 % (IX)
1,6 % Pentachlorbenzol
1,2 % (VIII)
3,2 % (XIV)
2,1 % (XII)

Beispiel 4

Das Beispiel 2 wurde wiederholt mit einer Reaktionszeit von nur 2 Stunden. Die gaschromatographische Untersuchung ergab folgende Gehalte im Umsetzungsgemisch:

60,0 % (II)
4,7 % (III)
5,1 % (IX)
2,5 % (XIV)
2,9 % (XII)
1,3 % Pentachlorbenzol
5,6 % (VIII)
2,5 % (I)

Beispiel 5

Das Beispiel 2 wurde mit der Ausnahme wiederholt, daß der Druck auf 100 mbar eingestellt wurde. Hierdurch ergab sich eine durch Siedekühlung kontrollierbare Innentemperatur von 120 ° C. Die gaschromatographische Analyse des Umsetzungsgemisches ergab folgende Gehalte:

69,2 % (II)
5,8 % (III)
5,6 % (IX)
2,4 % (XIV)
3,1 % (XII)
1,2 % Pentachlorbenzol
3,9 % (VIII)
0,7 % (I)

Beispiel 6

Es wurde wie im Beispiel 2 gearbeitet, die Reaktionszeit betrug jedoch nur 3 Stunden. Das KF wurde ohne weitere Vorbehandlung eingesetzt. Die gaschromatographische Analyse des Umsetzungsgemisches ergab folgende Gehalte:

61,5 % (II)
5,2 % (III)
5,1 % (IX)
2,6 % (XIV)
3,3 % (XII)
1,4 % Pentachlorbenzol
5,7 % (VIII)
2,2 % (I)

Die zuletzt beschriebenen Beispiele zeigen die Einstellung verschiedener Reinheitsgrade von (II) durch Variation der Reaktionsparameter.

Beispiel 7

Das Beispiel 5 wurde wiederholt. Die Umsetzungsprodukte wurden mit n-Hexan aus der Lösung im DMSO extrahiert, wie im Beispiel 1 beschrieben wurde. Ebenfalls gemäß obiger Beschreibung wurde das Umsetzungsgemisch in n-Hexan katalytisch hydriert; die gebildeten substituierten Aniline wurden als Hydrochlorid gefällt. Diese von der übrigen Lösung getrennten Anilinhydrochloride wurden mit Phosgen in die zugehörigen Isocyanate umgesetzt. Hierzu suspendierte man 90 g Anilinhydrochlorid-Gemisch, enthaltend 95,0 Gew.-% (0,37 Mol) (VI) und 4,9 Gew.-% (0,02 Mol) (X), in 400 ml Toluol und leitete bei Raumtemperatur 60 g (0,6 Mol) Phosgen ein, heizte langsam auf 100 °C und hielt diese Temperatur 5 Stunden. Nach dem Ausblasen des Phosgens mit einem $N_2$-Strom destillierte man das Toluol ab und trennte die so erhaltenen Isocyanate destillativ. Man erhielt bei 24 mbar und 112 °C destillativ das (VII). Nach Abkühlen des Destillationsrückstandes und weiterer Absenkung des Druckes auf 1 mbar war bei 50 °C der Beginn einer leichten Sublimation festzustellen. Bei 120 °C/1 mbar konnte sodann (XI) in befriedigender Weise durch Sublimation erhalten werden.

**Patentansprüche**

**1.** Verfahren zur Herstellung von substituierten 3,5-Dichlor-2,4-difluor-benzolender Formel

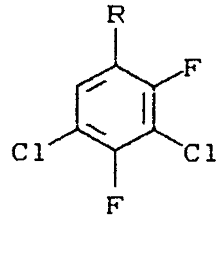

in der

R für Nitro, Amino (einschließlich der zugehörigen Ammoniumsalze) oder Isocyanato steht, aus 2,3,4,5-Tetrachlor-nitrobenzol durch zunächst durchgeführte Umsetzung mit Alkalifluoriden bei erhöhter Temperatur in einem polaren, aprotischen Lösungsmittel, dadurch gekennzeichnet, daß man ein rohes 2,3,4,5-Tetrachlor-nitrobenzol mit einem Gehalt von 2 bis 40 Gew.-% an 2,3,5,6-Tetrachlor-nitrobenzol und untergeordnete Mengen an 2,3,4,6-Tetrachlor-nitrobenzol, bezogen auf das gesamte Tetrachlor-nitrobenzol, einsetzt und das substituierte 3,5-Dichlor-2,4-difluor-benzol auf der Nitrostufe oder nach Reduktion der Nitrogruppe auf der Aminostufe oder nach Reduktion und Phosgenierung der Aminogruppe auf der Isocyanatstufe aus dem Reaktionsgemisch isoliert.

**2.** Verfahren nach Anspruch 1, dadurch gekennzeichnet, daß man die zunächst durchgeführte Umsetzung bei einer Temperatur von 60 - 160 °C, bevorzugt bei 80 bis 140 °C, besonders bevorzugt bei 95 bis 125 °C, und einer Reaktionszeit von 20 - 0,2 Stunden so durchführt, daß die Umwandlung des unerwünschten 2,2,5,6-Tetrachlor-nitrobenzols in 2,3,5,6-Tetrachlor-fluorbenzol und/oder des unerwünschten 2,3,4,6-Tetrachlor-nitrobenzols in 2,3,4,6-Tetrachlor-fluorbenzol bis zum gewünschten Grad an restlicher Verunreinigung erfolgt, das Reaktionsgemisch abkühlt und in die Alkalifluoride/-chloride, das Lösungsmittel und die Umsetzungsprodukte auftrennt und danach die Umsetzungsprodukte auf der Nitrostufe, der Aminostufe oder der Isocyanatstufe physikalisch von den Umwandlungsprodukten des 2,3,5,6-Tetrachlor-nitrobenzols und/oder des 2,3,4,6-Tetrachlor-nitrobenzols abtrennt.

**3.** Verfahren nach Ansprüchen 1 und 2, dadurch gekennzeichnet, daß als polares, aprotisches Lösungsmittel Dimethylsulfoxid (DMSO), Dimethylformamid (DMF) oder Dimethylsulfon ($DMSO_2$) eingesetzt wird.

**4.** Verfahren nach Ansprüchen 1 und 2, dadurch gekennzeichnet, daß als polares, aprotisches Lösungsmittel DMSO eingesetzt wird.

**5.** Verfahren nach Ansprüchen 1 bis 4, dadurch gekennzeichnet, daß als Alkalifluorid Kaliumfluorid eingesetzt wird.

**6.** Verfahren nach Ansprüchen 1 bis 5, dadurch gekennzeichnet, daß als polares, aprotisches Lösungsmittel DMSO eingesetzt wird und bei einer Temperatur von 60 bis 160 °C, bevorzugt 80 bis 140 °C, besonders bevorzugt 95 bis 125 °C und in einer Mindestreaktionszeit von 20 bis 0,2 Stunden gearbeitet wird, wobei die Temperatur und die Zeit durch die Beziehung T (°C) = A -33 log t (h) verknüpft sind, in der A Werte von 100-137, bevorzugt 110-135, besonders bevorzugt 115-130 annimmt.

**7.** Verfahren nach Ansprüchen 1 bis 6, dadurch gekennzeichnet, daß zur Auftrennung des Reaktionsgemisches das Alkalifluorid/-chlorid abgetrennt wird und dann die Umsetzungsprodukte aus dem polaren, aprotischen Lösungsmittel mit Hilfe eines oder mehrerer geradkettiger oder verzweigter offenkettiger oder cyclischer aliphatischer Kohlenwasserstoffe mit einem Siedepunkt von mindestens 30 °C extrahiert werden.

**8.** Verfahren nach Ansprüchen 1 bis 7, dadurch gekennzeichnet, daß der gewünschte Grad restlicher Verunreinigung nur zum Teil durch Umwandlung von 2,3,5,6-Tetrachlor-nitrobenzol in 2,3,5,6-Tetrachlor-fluorbenzol und/oder 2,3,4,6-Tetrachlornitrobenzol in 2,3,4,6-Tetrachlor-fluorbenzol eingestellt wird, die Umsetzungsprodukte zur Anilinstufe reduziert werden, die Aniline durch Salzbildung ausgefällt und abgetrennt werden und nach Rückverwandlung der Salze in die freien Aniline das 3,5-Dichlor-2,4-difluor-anilin durch destillative Abtrennung von Tetrachlor-anilinen erhalten wird.

**9.** Verfahren nach Ansprüchen 1 bis 7, dadurch gekennzeichnet, daß der gewünschte Grad restlicher Verunreinigung nur zum Teil durch Umwandlung von 2,3,5,6-Tetrachlor-nitrobenzol in 2,3,5,6-Tetrachlor-fluorbenzol und/oder 2,3,4,6-Tetrachlornitrobenzol in 2,3,4,6-Tetrachlor-fluorbenzol eingestellt wird, die Umsetzungsprodukte zur Anilinstufe reduziert werden, die Aniline durch Salzbildung ausgefällt und abgetrennt werden, die Anilinsalze als solche oder nach Rückverwandlung der Salze in die freien Aniline zur Isocyanatstufe phosgeniert werden und das 3,5-Dichlor-2,4-difluorphenylisocyanat durch destillative Abtrennung von Tetrachlor-phenylisocyanaten erhalten wird.

**Claims**

1. Process for the preparation of substituted 3,5-dichloro-2,4-difluoro-benzenesof the formula

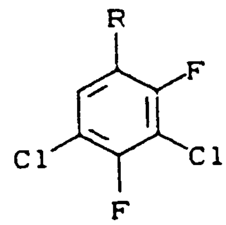

in which

R represents nitro, amino (including the corresponding ammonium salts) or isocyanato,

from 2,3,4,5-tetrachloro-nitrobenzene by means of the reaction, carried out initially, with alkali metal fluorides at elevated temperature in a polar, aprotic solvent, characterized in that a crude 2,3,4,5-tetrachloro-nitrobenzene containing 2 to 40% by weight of 2,3,5,6-tetrachloro-nitrobenzene and minor amounts of 2,3,4,6-tetrachloro-nitrobenzene, relative to the total amount of tetrachloro-nitrobenzene, is employed and the substituted 3,5-dichloro-2,4-difluorobenzene is isolated from the reaction mixture at the nitro stage or, after reduction of the nitro group, at the amino stage or, after reduction and phosgenation of the amino group, at the isocyanate stage.

2. Process according to Claim 1, characterized in that the reaction, carried out initially, is carried out at a temperature of 60 - 160 ° C, preferably 80 to 140 ° C, particularly preferably 95 to 125 ° C, and a reaction time of 23 - 0.2 hours in a fashion such that the undesired 2,3,5,6-tetrachloro-nitrobenzene is converted into 2,3,5,6-tetrachloro-fluorobenzene and/or the undesired 2,3,4,6-tetrachloro-nitrobenzene is converted into 2,3,4,6-tetrachloro-fluorobenzene to the desired degree of residual contamination, the reaction mixture is cooled and separated into the alkali metal fluorides/chlorides, the solvent and the reaction products, and the reaction products are then separated physically from the conversion products of 2,3,5,6-tetrachloro-nitrobenzene and/or 2,3,4,6-tetrachloro-nitrobenzene at the nitro stage, the amino stage or the isocyanate stage.

3. Process according to Claims 1 and 2, characterized in that the polar, aprotic solvent employed is dimethyl sulphoxide (DMSO), dimethylformamide (DMF) or dimethyl sulphone ($DMSO_2$).

4. Process according to Claims 1 and 2, characterized in that the polar, aprotic solvent employed is DMSO.

5. Process according to Claims 1 to 4, characterized in that the alkali metal fluoride employed is potassium fluoride.

6. Process according to Claims 1 to 5, characterized in that the polar, aprotic solvent employed is DMSO and the process is carried out at a temperature of 60 to 160 ° C, preferably 80 to 140 ° C, particularly preferably 95 to 125 ° C, and at a minimum reaction time of 20 to 0.2 hours, the temperature and the time being linked by the relationship T (° C) = A -33 log t (h), in which A assumes values of 100 - 137, preferably 110 - 135, particularly preferably 115 - 130.

7. Process according to Claims 1 to 6, characterized in that, in order to resolve the reaction mixture, the alkali metal fluoride/chloride is removed and the reaction products are then extracted from the polar, aprotic solvent with the aid of one or more straight-chain or branched open-chain or cyclic aliphatic hydrocarbons having a boiling point of at least 30 ° C.

8. Process according to Claims 1 to 7, characterized in that the desired degree of residual contamination is produced only partially by converting 2,3,5,6-tetrachloro-nitrobenzene into 2,3,5,6-tetrachloro-fluorobenzene and/or 2,3,4,6-tetrachloro-nitrobenzene into 2,3,4,6-tetrachloro-fluorobenzene, reducing the reaction products to the aniline stage, precipitating the anilines by salt formation and removing them, and, after reconverting the salts into the free anilines, obtaining 3,5-dichloro-2,4-difluoro-aniline by distillative separation of tetrachloro-anilines.

9. Process according to Claims 1 to 7, characterized in that the desired degree of residual contamination is only partly produced by converting 2,3,5,6-tetrachloronitrobenzene into 2,3,5,6-tetrachloro-fluorobenzene and/or 2,3,4,6-tetrachloro-nitrobenzene into 2,3,4,6-tetrachloro-fluorobenzene, reducing the reaction products to the aniline stage, precipitating the anilines by salt formation and removing them, phosgenating the aniline salts, as such or after reconversion of the salts into the free anilines, to the isocyanate stage, and obtaining 3,5-dichloro-2,4-difluoro-phenyl isocyanate by distillative separation of tetrachloro-phenyl isocyanates.

## Revendications

1. Procédé de production de 3,5-dichloro-2,4-difluoro-benzènes substitués de formule

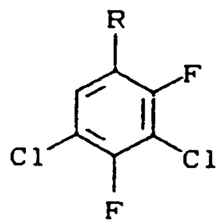

dans laquelle

R est un groupe nitro, amino (y compris les sels d'ammonium correspondants) ou isocyanato,

à partir de 2,3,4,5-tétrachloro-nitrobenzène par réaction préalable avec des fluorures alcalins à température élevée dans un solvant aprotique polaire, caractérisé en ce qu'on utilise un 2,3,4,5-tétrachloro-nitrobenzène brut ayant une teneur en 2,3,5,6-tétrachloro-nitrobenzène de 2 à 40 % en poids et contenant de faibles quantités de 2,3,4,6-tétrachloro-nitrobenzène, par rapport au tétrachloro-nitrobenzène total et on isole du mélange réactionnel le 3,5-dichloro-2,4-difluoro-benzène substitué, au stade nitro ou, après réduction du groupe nitro, au stade amino ou, après réduction et phosgénation du groupe amino, au stade isocyanate.

2. Procédé suivant la revendication 1, caractérisé en ce qu'on conduit la réaction préalable à une température de 60 à 160 °C, de préférence de 80 à 140 °C, notamment de 95 à 125 °C, et pendant une durée de réaction de 20 à 0,2 heures en effectuant la transformation du 2,3,5,6-tétrachloro-nitrobenzène non désiré en 2,3,5,6-tétrachloro-fluorobenzène et/ou du 2,3,4,6-tétrachloro-nitrobenzène non désiré en 2,3,4,6-tétrachloro-fluorobenzène jusqu'au degré désiré d'impureté résiduelle, on refroidit le mélange réactionnel et on sépare les fluorures/chlorures alcalins, le solvant et les produits de réaction puis on isole physiquement ces derniers au stade nitro, au stade amino ou au stade isocyanate, des produits de transformation du 2,3,5,6-tétrachloro-nitrobenzène et/ou du 2,3,4,6-tétrachloro-nitrobenzène.

3. Procédé suivant les revendications 1 et 2, caractérisé en ce qu'on utilise comme solvant aprotique polaire le diméthylsulfoxyde (DMSO), le diméthylformamide (DMF) ou la diméthylsulfone ($DMSO_2$).

4. Procédé suivant les revendications 1 et 2, caractérisé en ce qu'on utilise le DMSO comme solvant aprotique polaire.

5. Procédé suivant les revendications 1 à 4, caractérisé en ce qu'on utilise le fluorure de potassium comme fluorure alcalin.

6. Procédé suivant les revendications 1 à 5, caractérisé en ce qu'on utilise le DMSO comme solvant aprotique polaire et on opère à une température de 60 à 160 °C, de préférence de 80 à 140 °C, notamment de 95 à 125 °C, et pendant une durée minimale de réaction de 20 à 0,2 heures, la température et le temps étant liés par la relation $T\ (°C) = A - 33 \log t\ (h)$ dans laquelle A prend des valeurs de 100 à 137, de préférence de 110 à 135, notamment de 115 à 130.

7. Procédé suivant les revendications 1 à 6, caractérisé en ce que pour diviser le mélange réactionnel, on sépare le fluorure/chlorure alcalin puis on extrait les produits de réaction du solvant aprotique polaire à

l'aide d'un ou plusieurs hydrocarbures aliphatiques à chaîne ouverte droite ou ramifiée, ou cycliques, ayant un point d'ébullition d'au moins 30 ° C.

**8.** Procédé suivant les revendications 1 à 7, caractérisé en ce que le degré désiré d'impureté résiduelle n'est que partiellement établi par transformation du 2,3,5,6-tétrachloro-nitrobenzène en 2,3,5,6-tétrachloro-fluorobenzène et/ou du 2,3,4,6-tétrachloro-nitrobenzène en 2,3,4,6-tétrachloro-fluorobenzène, on réduit les produits de réaction au stade d'aniline, on précipite les anilines par formation de sels et on les sépare et, après rétrogradation des sels en les anilines libres, on obtient la 3,5-dichloro-2,4-difluoraniline par séparation de tétrachlor-anilines par distillation.

**9.** Procédé suivant les revendications 1 à 7, caractérisé en ce que le degré désiré d'impureté résiduelle n'est que partiellement établi par transformation du 2,3,5,6-tétrachloro-nitrobenzène en 2,3,5,6-tétrachloro-fluorobenzène et/ou du 2,3,4,6-tétrachloro-nitrobenzène en 2,3,4,6-tétrachloro-fluorobenzène, les produits de réaction sont réduits au stade d'aniline, les anilines sont précipitées par formation de sels et séparées, les sels d'anilines sont phosgénés au stade isocyanate tels quels ou après rétrogradation en les anilines libres et le 3,5-dichloro-2,4-difluophénylisocyanate est obtenu par séparation par distillation de tétrachloro-phényliso-cyanates.